# FASCICULE DE BREVET EUROPEEN

(11) **EP 3 645 578 B1**
(45) Date de publication et mention de la délivrance du brevet: **06.09.2023**
(21) Numéro de dépôt: 18749013.1
(22) Date de dépôt: 27.06.2018
(51) Int. Cl.: C08C 19/22, B60C 1/00, C08F 8/30, C08L 15/00, C07D 233/00, C07D 233/64, C08C 19/00, C08C 19/10

(54) **ELASTOMÈRE DIÉNIQUE PORTANT DES GROUPES IMIDAZOLE**
DIENKAUTSCHUK MIT IMIDAZOLGRUPPEN
DIENE RUBBER BEARING IMIDAZOLE GROUPS

(30) Priorité: 28.06.2017 FR 1755940
(43) Date de publication de la demande: 06.05.2020
(73) Titulaire: Compagnie Générale des Etablissements Michelin, 63000 Clermont-Ferrand (FR)
(72) Inventeur: SCHNELL, Benoît, 63040 Clermont-Ferrand Cedex 9 (FR); LEDOUX, Céline, 63040 Clermont-Ferrand Cedex 9 (FR); MAISONNEUVE, Lise, 63040 Clermont-Ferrand Cedex 9 (FR); DRONET, Séverin, 63040 Clermont-Ferrand Cedex 9 (FR)
(74) Mandataire: Gandon-Pain, Sylvie
(86) Numéro de dépôt international: PCT/FR2018/051564
(87) Numéro de publication internationale: WO 2019/002756

(56) Documents cités:
- WO-A1-2015/059269
- WO-A1-2015/059271
- WO-A1-2017/121950
- FR-A1- 3 046 603
- JP-A- 2015 044 929

## Description

La présente invention concerne le domaine des élastomères diéniques modifiés, notamment utilisables dans des compositions de caoutchouc pour pneumatique.

Une des exigences requises pour un pneumatique est d'assurer une adhérence optimale sur route, notamment sur sol mouillé. Une façon de conférer au pneumatique une adhérence élevée sur sol mouillé est d'utiliser une composition de caoutchouc dans sa bande de roulement, laquelle composition présente un large potentiel hystérétique.

Mais en même temps la bande de roulement de pneumatique doit aussi minimiser sa contribution à la résistance au roulement du pneumatique, c'est à dire être la moins hystérétique possible. La composition de caoutchouc de la bande de roulement doit donc satisfaire à deux exigences antinomiques, à savoir présenter un potentiel d'hystérèse maximal pour satisfaire à l'exigence d'adhérence et présenter une hystérèse aussi faible que possible pour satisfaire à l'exigence de résistance au roulement. Satisfaire à la fois à l'exigence d'adhérence, notamment sur sol mouillé, et de résistance au roulement reste une préoccupation constante des manufacturiers de pneumatiques.

Pour réduire la résistance au roulement d'une bande de roulement de pneumatique, il a été proposé d'utiliser des élastomères diéniques portant des cycles imidazoles comme groupes pendants, mais à un taux molaire d'au plus 3%. On peut par exemple se référer à la publication de la demande de brevet WO 2015059271.

La Demanderesse a découvert que le compromis de performance d'un pneumatique entre l'adhérence et la résistance au roulement est amélioré lorsque l'élastomère diénique contenu dans la composition de caoutchouc de la bande de roulement comprend des groupes pendants imidazole répartis de façon aléatoire le long de la chaîne de l'élastomère à taux molaire bien supérieur à 3%.

Ainsi, un premier objet de l'invention est un élastomère diénique qui porte des cycles imidazole de formule (I) pendants et répartis de façon aléatoire le long de la chaîne de l'élastomère diénique, 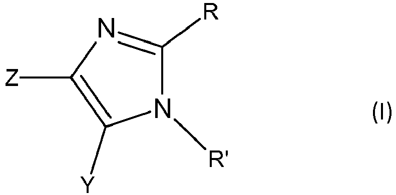 dans laquelle :
trois des quatre symboles Z, Y, R et R', identiques ou différents, représentent un atome d'hydrogène ou une chaîne carbonée pouvant contenir au moins un hétéroatome, Z et Y pouvant former ensemble avec les atomes de carbone auxquels ils se rattachent un cycle lorsque ni Z, ni Y ne désignent le 4^{ème} symbole,
et le seul quatrième symbole désigne un rattachement à l'élastomère diénique,
caractérisé en ce que les cycles imidazoles représentent entre 4 et 15 moles pour 100 moles d'unités monomères constituant l'élastomère diénique.

Un autre objet de l'invention est une composition de caoutchouc qui comprend une charge renforçante et l'élastomère diénique conforme à l'invention.

L'invention concerne aussi une bande de roulement qui comprend la composition de caoutchouc conforme à l'invention.

L'invention concerne également un pneumatique qui comprend une bande de roulement conforme à l'invention.

### 1. DESCRIPTION DETAILLEE DE L'INVENTION

Tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents).

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont concernés notamment les élastomères, les plastifiants, les charges ...

L'élastomère diénique conforme à l'invention a pour caractéristique essentielle de contenir à la fois des unités diéniques et des cycles imidazoles. Il est considéré comme un élastomère diénique fonctionnalisé. Par unité diénique, on entend une unité qui résulte de l'insertion d'un monomère diène (monomères porteurs de deux doubles liaisons carbone-carbone, conjuguées ou non) par polymérisation dans une chaîne polymère et qui contient une double liaison carbone carbone. Le monomère diène est de préférence un 1,3-diène, notamment le 1,3-butadiène ou l'isoprène, auquel cas l'élastomère diénique a pour unité diénique des unités monomères 1,3-butadiène ou 1,3-isoprène, de préférence 1,3-butadiène.

De préférence l'élastomère diénique dérive d'un élastomère choisi dans le groupe constitué par les polybutadiènes, les polyisoprènes, les copolymères de butadiène, les copolymères d'isoprène et les mélanges de ces élastomères. De manière plus préférentielle, l'élastomère diénique dérive d'un élastomère de synthèse (ou élastomère synthétique). En d'autres termes, l'élastomère diénique conforme à l'invention ne dérive pas d'un caoutchouc naturel. On rappelle que le caoutchouc naturel traditionnellement utilisé comme élastomère dans les compositions de caoutchouc provient de la matière sèche caoutchouteuse du latex de caoutchouc naturel, très souvent extraite de l'hévéa et n'est donc pas considéré comme un élastomère de synthèse.

Les cycles imidazoles portés par l'élastomère diénique conforme à l'invention comme groupes pendants le long de la chaîne de l'élastomère diénique répondent à la formule (I) 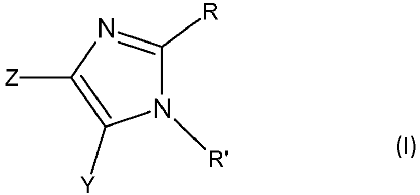 dans laquelle :
trois des quatre symboles Z, Y, R et R', identiques ou différents, représentent un atome d'hydrogène ou une chaîne carbonée pouvant contenir au moins un hétéroatome, Z et Y pouvant former ensemble avec les atomes de carbone auxquels ils se rattachent un cycle lorsque ni Z, ni Y ne désigne le 4^{ème} symbole,
et le seul quatrième symbole désigne un rattachement à l'élastomère diénique.

Selon un mode de réalisation, Z et Y sont chacun un atome d'hydrogène.

Selon un autre mode de réalisation, R représente un atome d'hydrogène ou une chaîne carbonée pouvant contenir au moins un hétéroatome.

Selon un mode de réalisation préférentiel de l'invention, R' désigne le rattachement, direct ou indirect, à l'élastomère, auquel cas R' est le 4^{ème} symbole. Selon ce mode de réalisation de l'invention, le cycle imidazole présente de préférence l'une des caractéristiques suivantes ou de manière plus préférentielle les deux :
- Z et Y sont chacun un atome d'hydrogène,
- R représente un atome d'hydrogène ou une chaîne carbonée pouvant contenir au moins un hétéroatome.

Lorsque R représente une chaîne carbonée pouvant contenir au moins un hétéroatome, R contient préférentiellement 1 à 6 atomes de carbone. A titre de chaîne carbonée, conviennent notamment les groupes alkyles, tout particulièrement le groupe méthyle.

Les groupes pendants utiles aux besoins de l'invention sont répartis de façon aléatoire le long de la chaîne de l'élastomère diénique conforme à l'invention. L'élastomère diénique conforme à l'invention ne doit pas être confondu avec les élastomères diéniques décrits dans le brevet US 5,346,962. En effet les élastomères de l'art antérieur comportent des cycles imidazoles qui sont présents dans la chaîne élastomère uniquement sous la forme de bloc polyvinylimidazole ou polyvinylbenzimidazole et en aucun cas, ils ne comportent des cycles imidazoles répartis de façon aléatoire le long de leur chaîne. Avantageusement, l'élastomère diénique conforme à l'invention est dépourvu de tout bloc polyvinylimidazole, l'imidazole étant substitué ou non substitué, ou est dépourvu de tout bloc polyvinylbenzimidazole, le benzimidazole étant substitué ou non substitué.

Un élastomère a typiquement une masse molaire moyenne en masse d'au moins 80000 g/mol, préférentiellement d'au moins 100000 g/mol. Un élastomère n'est pas à confondre avec un polymère liquide dont la masse molaire moyenne en masse est insuffisante pour conférer des propriétés élastiques à la chaîne polymère. Les polymères diéniques liquides sont généralement des plastifiants.

Les élastomères diénique qui portent des cycles imidazoles de formule (la) le long de la chaîne de l'élastomère diénique dans laquelle le symbole * désigne un rattachement direct ou indirect à l'élastomère, peuvent être synthétisés par copolymérisation, notamment en émulsion par voie radicalaire, d'au moins un diène et du vinylimidazole. Par exemple peuvent être cités des copolymères comprenant des unités diéniques, notamment 1,3-butadiène, et des unités N-vinylimidazole.

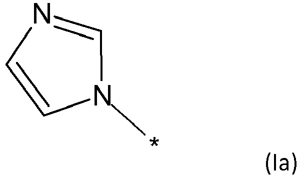

Les élastomères diénique qui portent des cycles imidazoles de formule (Ib) le long de la chaîne de l'élastomère diénique dans laquelle le symbole * désigne un rattachement direct ou indirect à l'élastomère, R étant comme défini précédemment, peuvent être synthétisés par copolymérisation, notamment en émulsion par voie radicalaire, d'au moins un diène et un monomère vinylimidazole, le cycle imidazole étant substitué en position 2. Par exemple peuvent être cités des copolymères comprenant des unités diéniques, notamment 1,3-butadiène, et des unités N-vinyl-2-méthylimidazole.

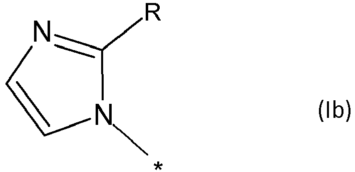

Selon un mode de réalisation de l'invention, les cycles imidazoles sont greffés sur les unités diéniques de l'élastomère diénique. Autrement dit, le point de rattachement de chaque cycle imidazole à l'élastomère diénique se fait par greffage sur une unité diénique.

Selon un mode de réalisation préférentiel de l'invention, les cycles imidazoles sont greffés sur l'élastomère diénique par réaction d'un composé 1,3-dipolaire et d'un élastomère diénique de départ. Le composé 1,3-dipolaire répond à la formule (II)

Q-A-B (II)

dans laquelle
Q comprend un dipôle contenant un atome d'azote,
A est un espaceur reliant Q et B,
B représente le cycle imidazole de formule (I) dans laquelle le quatrième symbole est rattaché directement à A.

Le terme composé 1,3-dipolaire est compris selon la définition donnée par IUPAC.

L'élastomère diénique de départ est tout élastomère diénique, c'est-à-dire tout élastomère constitué au moins en partie (i.e., un homopolymère ou un copolymère) d'unités monomères diènes. Dans l'objectif de synthétiser l'élastomère conforme à l'invention qui contient à la fois des unités diéniques et des cycles imidazoles pendants, l'homme du métier comprend que la fraction molaire d'unité diénique dans l'élastomère diénique de départ est supérieure à la valeur visée de la fraction molaire de cycles imidazoles que l'on souhaite greffer sur l'élastomère diénique.

Ces définitions étant données, on entend plus particulièrement par élastomère diénique susceptible d'être utilisé dans les compositions conformes à l'invention :
(a) - tout homopolymère d'un monomère diène conjugué, notamment tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyles aromatiques ayant de 8 à 20 atomes de carbone;
(c) - tout copolymère ternaire obtenu par copolymérisation d'éthylène, d'une a-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - tout copolymère d'isobutène et d'isoprène (caoutchouc butyle), ainsi que les versions halogénées, en particulier chlorées ou bromées, de ce type de copolymère ;
(e) - tout copolymère obtenu par copolymérisation d'un ou plusieurs diènes conjugués avec l'éthylène, une a-monooléfine ayant 3 à 18 atomes de carbone ou leur mélange comme par exemple ceux décrits dans le document WO 2005028526, WO 2004035639 et WO 2007054224.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène. A titre de composés vinylaromatique conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène. A titre d'α-monooléfines aliphatiques acycliques ayant 3 à 18 atomes de carbone, on peut citer le propène, le butène, l'hexène, l'octène, l'hexadécène.

Préférentiellement, l'élastomère diénique de départ est choisi dans le groupe constitué par les polybutadiènes, les polyisoprènes, les copolymères de butadiène, les copolymères d'isoprène et leurs mélanges. Plus préférentiellement, l'élastomère diénique de départ est un élastomère de synthèse (ou élastomère synthétique). En d'autres termes, l'élastomère diénique de départ n'est pas un caoutchouc naturel. Encore plus préférentiellement, l'élastomère diénique de départ est choisi dans le groupe constitué par les copolymères de butadiène-styrène (SBR), les copolymères d'isoprène-butadiène (BIR), les copolymères d'isoprène-styrène (SIR), les copolymères d'isoprène-butadiène-styrène (SBIR) et les copolymères d'éthylène et de butadiène.

Le greffage du composé 1,3-dipolaire peut être réalisé en masse, par exemple dans un mélangeur interne ou un mélangeur externe tel qu'un mélangeur à cylindres. Le greffage est alors mis en oeuvre soit à une température du mélangeur externe ou du mélangeur interne inférieure à 60°C, suivi d'une étape de réaction de greffage sous presse ou en étuve à des températures allant de 80°C à 200°C, soit à une température du mélangeur externe ou du mélangeur interne supérieure à 60°C sans traitement thermique postérieur. Lorsque le greffage est réalisé en masse, il est réalisé préférentiellement en présence d'un antioxydant.

Le procédé de greffage peut également être effectué en solution en continu ou en discontinu. L'élastomère diénique ainsi modifié peut être séparé de sa solution par tout type de moyen connu par l'homme de l'art et en particulier par une opération de stripping à la vapeur d'eau.

Selon un mode de réalisation préférentiel de l'invention, le composé 1,3-dipolaire est choisi dans le groupe constitué par les oxydes de nitrile, les imines de nitrile et les nitrones. Le composé 1,3-dipolaire est alors tel que le symbole Q contient un motif -C=N→O, -C=N→N- ou -C=N(->O)-.

Le greffage du composé 1,3-dipolaire est effectué par cycloaddition [3+2] du ou des groupes réactifs du composé 1,3-dipolaire sur une ou plusieurs doubles liaisons d'une chaîne élastomère diénique. Le mécanisme de la cycloaddition d'un oxyde de nitrile, d'une nitrone et d'une imine de nitrile peut être illustré par les équations suivantes, dans lesquelles le symbole 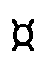 représente un quelconque substituant :
- Cycloaddition d'un oxyde de nitrile sur une insaturation ou double liaison d'un élastomère diénique (ici un polyisoprène) 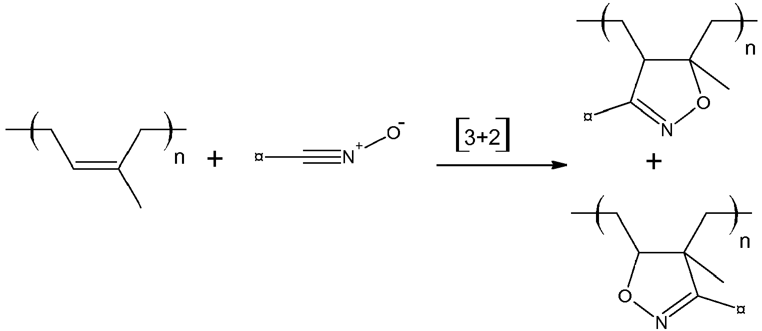
- Cycloaddition d'une nitrone sur une insaturation ou double liaison d'un élastomère diénique (ici un polyisoprène) 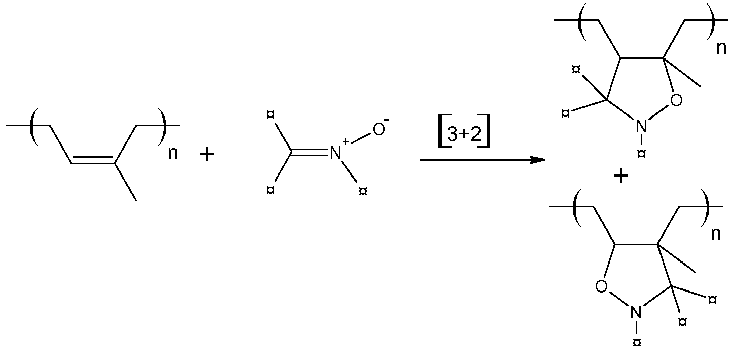
- Cycloaddition d'une imine de nitrile sur une insaturation ou double liaison d'un élastomère diénique (ici un polyisoprène)

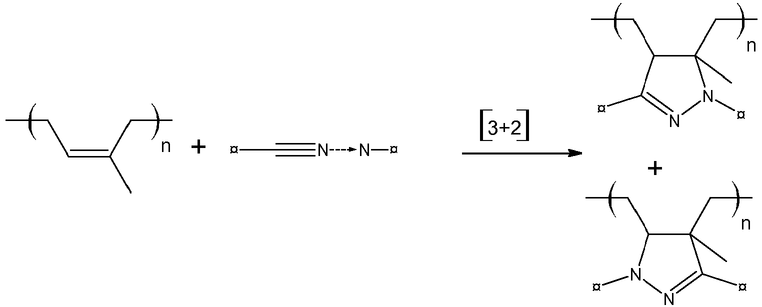

De préférence, le composé 1,3-dipolaire est tel que le symbole Q contient un motif C≡N→O. De manière plus préférentielle, le symbole Q comprend un noyau benzénique substitué par le dipôle oxyde de nitrile et également substitué par un ou plusieurs autres substituants dont un est en ortho du dipôle oxyde de nitrile.

De manière encore plus préférentielle, le symbole Q représente le motif répondant à la formule (III) dans laquelle quatre des cinq symboles R1 à R5 identiques ou différents, représentent un atome ou un groupe d'atomes, préférentiellement un groupe aliphatique ou un groupe aromatique et le cinquième symbole désigne un rattachement direct à A, sachant que R1 et R5 sont tous les deux différents de H.

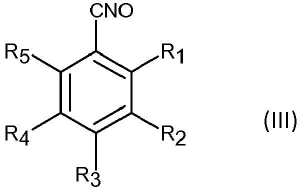

Les symboles R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle. Les symboles R2 et R4 sont préférentiellement chacun un atome d'hydrogène.

A est de préférence une chaîne carbonée pouvant contenir au moins un hétéroatome, laquelle chaîne carbonée contient de préférence 1 à 6 atomes de carbone, en particulier 1 à 3 atomes de carbone. De manière plus préférentielle, A est un groupe alcanediyle, de préférence méthanediyle.

Avantageusement, le composé 1,3-dipolaire utile aux besoins de l'invention est le composé 2,4,6-triméthyl-3-((2-méthyl-1*H*-imidazol-1-yl)méthyl)benzo-nitrile oxyde de formule (III-a) ou le composé 2,4,6-triéthyl-3-((2-méthyl-1*H*-imidazol-1-yl)méthyl)benzo-nitrile oxyde de formule (III-b) .

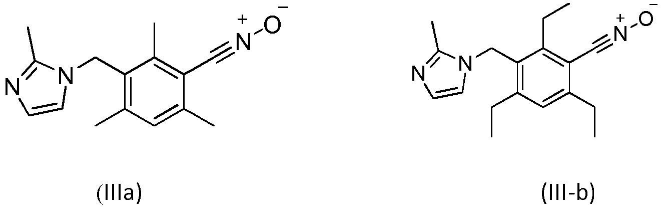

Le taux de cycles imidazoles pendants le long de la chaîne de l'élastomère diénique est compris entre 4 et 15 moles pour 100 moles d'unités monomères constituant l'élastomère diénique. A un taux inférieur à 4% molaire, la quantité de cycles imidazoles pendants et répartis de façon aléatoire le long de la chaîne élastomère est insuffisante pour conférer au pneumatique le compromis recherché de performance entre l'adhérence et la résistance au roulement d'un pneumatique. A un taux supérieur à 15% molaire, la quantité de cycles imidazoles pendants et répartis de façon aléatoire le long de la chaîne élastomère est trop importante et la température de transition vitreuse devient alors trop élevée, notamment proche de la température ambiante, ce qui ôte l'intérêt d'utiliser l'élastomère diénique dans une composition de caoutchouc pour pneumatique. De préférence, ce taux varie de 5 à 15 moles pour 100 moles d'unités monomères constituant l'élastomère diénique.

L'élastomère conforme à l'invention peut être utilisé dans une composition de caoutchouc. La composition de caoutchouc a pour caractéristique essentielle de comprendre l'élastomère diénique conforme à l'invention et une charge renforçante.

La charge renforçante peut être tout type de charge dite renforçante, connue pour ses capacités à renforcer une composition de caoutchouc utilisable pour la fabrication de pneumatiques, par exemple une charge organique telle que du noir de carbone, une charge inorganique renforçante telle que de la silice à laquelle est associé de manière connue un agent de couplage, ou encore un mélange de ces deux types de charge. Une telle charge renforçante consiste typiquement en des nanoparticules dont la taille moyenne (en masse) est inférieure au micromètre, généralement inférieure à 500 nm, le plus souvent comprise entre 20 et 200 nm, en particulier et plus préférentiellement comprise entre 20 et 150 nm. La charge renforçante peut être utilisée à un taux compris entre 30 et 200 pce.

La composition de caoutchouc peut contenir un élastomère autre que celui conforme à l'invention, notamment un élastomère diénique traditionnellement utilisé dans les compositions de caoutchouc. La composition de caoutchouc comprend préférentiellement plus de 50 pce, plus préférentiellement plus de 80 pce de l'élastomère conforme à l'invention défini selon l'un quelconque des modes de réalisation de l'invention.

La composition de caoutchouc peut contenir un système de réticulation de l'élastomère. Le système de réticulation peut être un système de vulcanisation ou être à base d'un ou plusieurs composés peroxydes, par exemple conventionnellement utilisés dans les compositions de caoutchouc utilisables pour la fabrication de pneumatique. Le système de réticulation est préférentiellement un système de vulcanisation, c'est-à-dire un système à base de soufre (ou d'un agent donneur de soufre) et d'un accélérateur primaire de vulcanisation. A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine), ou encore des retardateurs de vulcanisation connus. Le soufre est utilisé à un taux préférentiel compris entre 0,5 et 12 pce, en particulier entre 1 et 10 pce. L'accélérateur primaire de vulcanisation est utilisé à un taux préférentiel compris entre 0,5 et 10 pce, plus préférentiellement compris entre 0,5 et 5,0 pce.

La composition de caoutchouc peut contenir en outre d'autres additifs connus pour être utilisés dans des compositions de caoutchouc pour pneumatiques, tels que des plastifiants, des anti-ozonants, des antioxydants.

La composition de caoutchouc conforme à l'invention est typiquement fabriquée dans des mélangeurs appropriés, en utilisant deux phases de préparation successives bien connues de l'homme du métier : une première phase de travail ou malaxage thermomécanique (phase dite « non-productive ») à haute température, jusqu'à une température maximale comprise entre 130°C et 200°C, suivie d'une seconde phase de travail mécanique (phase dite « productive ») jusqu'à une plus basse température, typiquement inférieure à 110°C, par exemple entre 40°C et 100°C, phase de finition au cours de laquelle est incorporé le système de réticulation.

La composition de caoutchouc conforme à l'invention, pouvant être soit à l'état cru (avant réticulation ou vulcanisation), soit à l'état cuit (après réticulation ou vulcanisation), peut être utilisée dans un article semi-fini pour pneumatique. En particulier, la composition de caoutchouc peut être utilisée comme bande de roulement pour pneumatique, autre objet de l'invention.

Le pneumatique selon l'invention a pour caractéristique essentielle de comprendre une composition de caoutchouc conforme à l'invention dans sa bande de roulement. Le compromis de performance entre l'adhérence et la résistance au roulement est amélioré, en particulier l'adhérence est améliorée sans l'être au détriment de la résistance au roulement.

Les caractéristiques précitées de la présente invention, ainsi que d'autres, seront mieux comprises à la lecture de la description suivante de plusieurs exemples de réalisation de l'invention, donnés à titre illustratif et non limitatif.

### Il. EXEMPLES DE REALISATION DE L'INVENTION

### 11.1-Mesures et tests utilisés :

a. Analyse RMN (résonance magnétique nucléaire) :
   Les spectres sont acquis sur un spectromètre 500 MHz BRUKER équipé d'une « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans le disulfure de carbone (CS2).
b. Analyse des propriétés mécaniques dynamiques :
   La propriété dynamique tan(δ) est mesurée sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99.
   On effectue un balayage en amplitude de déformation de 0,1% à 50% crête-crête (cycle aller), puis de 50% à 0,1% crête-crête (cycle retour). Les résultats exploités sont le facteur de perte tan(δ). L'effet Payne calculé comme la différence la valeur de tan(δ) maximale entre 0,1% et 50% de déformation (cycle retour) et la valeur de tan(δ) à 0,1% de déformation (cycle retour).
c. Méthode de mesure du coefficient de frottement (µ) :
   Les mesures de coefficient de frottement dynamique ont été réalisées selon une méthode identique à celle décrite par L. Busse, A. Le Gal, et M. Küppel (Modelling of Dry and Wet Friction of Silica Filled Elastomers on Self-Affine Road Surfaces, Elastomere Friction, 2010, 51, p. 8). Les éprouvettes sont réalisées par moulage, puis réticulation d'un support caoutchouteux carré (50mmx50mm) de 6 mm d'épaisseur. Après fermeture du moule, celuici est placé dans une presse à plateaux chauffants à la température (typiquement 150°C), et pendant le temps nécessaires à la réticulation du matériau (typiquement une heure), à une pression de 16 bars. Le sol utilisé pour réaliser ces mesures est une carotte prélevée sur un sol routier réel en béton bitumineux de type BBTM (norme NF P 98-137). Pour éviter les phénomènes de démouillage et l'apparition de forces d'adhésion parasites entre le sol et le matériau, le système sol+éprouvette est immergé dans une solution aqueuse à 5% d'un agent tensio-actif (Sinnozon - numéro CAS : 25155-30-0). La température de la solution aqueuse est régulée à l'aide d'un bain thermostatique. L'éprouvette est soumise à un mouvement de glissement en translation parallèlement au plan du sol. La vitesse de glissement Vg est fixée à 0,3 m/sec. La contrainte normale appliquée σₙ est de 300 kPa. Ces conditions sont décrites ci-après par « conditions de sol mouillé ». On mesure en continu la contrainte tangentielle σₜ opposée au mouvement de l'éprouvette sur le sol. Le rapport entre la contrainte tangentielle σₜ et la contrainte normale σₙ donne le coefficient de frottement dynamique µ. Les coefficients de frottement dynamiques sont mesurés pour une température de solution aqueuse de 10 et 45°C, obtenues en régime permanent après stabilisation de la valeur de la contrainte tangentielle σₜ.

Pour comparer des compositions avec des rigidités différentes, les valeurs indiquées dans les exemples sont les coefficients de frottements µ à 30°C de chaque composition normalisés par rapport à la valeur maximale de µ de chaque composition entre 10 et 45°C.

### 11.2 Préparation des élastomères modifiés et des compositions de caoutchouc :

Trois copolymères de 1,3-butadiène et de styrène (SBR), respectivement SBR1, SBR2 et SBR3, qui ont été synthétisés de façon conventionnelle en solution par polymérisation anionique, sont utilisés comme élastomères de départ pour préparer les élastomères modifiés. SBR1 est à 27% en masse de styrène et de Tg -48°C ; SBR2 est à 14% en masse de styrène et de Tg -66°C ; SBR3 est à 2% en masse de styrène et de Tg -88°C.

SBR4 et SBR5 sont préparés à partir de SBR1 ; SBR6 est préparé à partir de SBR2 ; SBR7 est préparé à partir de SBR3.

L'oxyde de nitrile aromatique utilisé pour modifier les élastomères de départ est l'oxyde de nitrile de formule suivante :

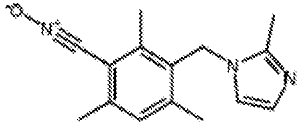

Les quantités d'oxyde de nitrile introduites sont respectivement de 10 pce et 20 pce pour préparer SBR4 et SBR5, elles sont de 40 pce pour préparer respectivement SBR6 et SBR7.

### Chaque élastomère modifié est préparé selon le mode opératoire suivant :

L'élastomère de départ et l'oxyde de nitrile sous la forme d'un mélange maître (en anglais « masterbatch ») sont introduits dans un mélangeur interne ThermoHaake muni de rotors de type Came et d'une cuve d'un volume de 85 cm3 régulée à 100°C et sont malaxés pendant 2 minutes à une vitesse de 60 tours par minute. L'élastomère modifié est caractérisé par analyse RMN pour déterminer le taux de cycle imidazole greffé au polymère. Le mélange maître est préparé par une mise en solution de l'élastomère et de l'oxyde de nitrile dans le tétrahydrofurane, le dichlorométhane ou le chloroforme, suivie d'une évaporation du solvant.

Ensuite, pour préparer chacune des compositions, au polymère modifié sont introduits les autres ingrédients de la composition de caoutchouc à l'exception du système de vulcanisation. On conduit alors un travail thermomécanique de 4 minutes à une vitesse de 60 tours par minute (phase non-productive), on récupère le mélange ainsi obtenu, on le refroidit, puis on incorpore du soufre et un accélérateur type sulfénamide sur un mélangeur (homo-finisseur) à 40 °C, en mélangeant le tout (phase productive) pendant un temps approprié (par exemple 5 à 10 minutes).

Ainsi sont préparées 4 compositions, C1 à C4 : la composition C1 contient l'élastomère diénique modifié SBR4 obtenu par modification du SBR1 par 10 pce d'oxyde de nitrile ; la composition C2 contient l'élastomère diénique modifié SBR5 obtenu par modification du SBR1 par 20 pce d'oxyde de nitrile ; la composition C3 contient l'élastomère diénique modifié SBR6 obtenu par modification du SBR2 par 40 pce d'oxyde de nitrile ; la composition C4 contient l'élastomère diénique modifié SBR7 obtenu par modification du SBR3 par 40 pce d'oxyde de nitrile. Le taux de cycles imidazoles est pour SBR4 inférieur à 4 moles pour 100 moles d'unités monomères constituant l'élastomère diénique ; il est compris entre 4 moles et 15 moles pour 100 moles d'unités monomères constituant l'élastomère diénique pour SBR5, SBR6 et SBR7.

Les compositions ainsi obtenues sont ensuite calandrées sous la forme de plaques (épaisseur de 2 à 3 mm) et les éprouvettes pour réaliser les analyses mécaniques dynamiques et les mesures des coefficients de frottement sont cuites pendant 60 minutes à 150°C sous presse.

Le détail des formulations des compositions de caoutchouc figure dans le tableau 1. Pour mettre en évidence l'influence du taux d'oxyde de nitrile greffé à l'élastomère (compositions C1 à C4), les quantités des ingrédients introduits dans la composition de caoutchouc sont indexées pour 100 parties d'élastomère non modifié. Pour les compositions C1 à C4, la quantité d'élastomères modifiés introduite dans la composition de caoutchouc est donc décrite dans le tableau 2 de façon à faire apparaître les deux composantes d'origine avec leur quantité respective que sont l'élastomère de départ (100 pce, élastomère non modifié) et l'oxyde de nitrile (quantité introduite en pce pour modifier l'élastomère de départ).

**Tableau 1**

| Composition | C1 | C2 | C3 | C4 |
|---|---|---|---|---|
| SBR4 | 110 | - | - | - |
| SBR5 | - | 120 | - | - |
| SBR6 | - | - | 140 | - |
| SBR7 | - | - | - | 140 |
| Antioxydant (1) | 1,5 | 1,5 | 1,5 | 1,5 |
| ZnO | 3 | 3 | 3 | 3 |
| Acide Stéarique | 2 | 2 | 2 | 2 |
| Soufre | 1 | 1 | 1 | 1 |
| CBS (2) | 2 | 2 | 2 | 2 |

| | | | | |
|---|---|---|---|---|
| (1) Antioxydant N-(1,3-dimétyl-butyl)-N'-phényl-p-phénylènediamine (2) N-cyclohexyl-2-benzothiazyl-sulfénamide (« Santocure CBS » de la société Flexsys) | | | | |

Les résultats de caractérisation mécaniques et de frottement de ces compositions sont présentés dans le tableau 2.

**Tableau 2**

| | C1 (comparatif) | C2 | C3 | C4 |
|---|---|---|---|---|
| Tan(δ)max-tan(δ)0,1% à 23°C | <0,01 | <0,01 | <0,01 | <0,01 |
| µ(30°C)/µmax | 0,84 | 0,93 | 0,96 | 1 |

La différence entre tan(δ)max et tan(δ) à 0,1% est représentative de l'effet Payne d'un matériau et dans le cas présent de la résistance au roulement : plus la valeur de (tan(δ) max - tan(δ)0,1%) est faible, plus la résistance au roulement est basse. Les 4 compositions présentent un effet Payne très faible.

La méthode de mesure du coefficient de frottement µ d'une composition de caoutchouc réalisée sur éprouvette est ici représentative de la performance d'adhérence sur sol mouillé d'un pneumatique dont la bande de roulement serait constituée de cette composition de caoutchouc. Elle permet d'obtenir un bon descripteur de la performance de freinage sur sol mouillé des matériaux pour une bande de roulement. On peut par exemple se référer à la demande de brevet WO 2016001052 A1.

Les compositions C2, C3 et C4 présente un coefficient de frottement à 30°C amélioré par rapport à C1 de 10 à 20%. Les compositions de caoutchouc qui contiennent les élastomères conformes à l'invention (C2, C3 et C4) présentent donc un compromis de performance entre l'adhérence et la résistance au roulement amélioré. Les élastomères modifiés conformes à l'invention confèrent aux compositions de caoutchouc les contenant une amélioration de ce compromis.

## Revendications

1. Elastomère diénique qui porte des cycles imidazole de formule (I) pendants et répartis de façon aléatoire le long de la chaîne de l'élastomère diénique, 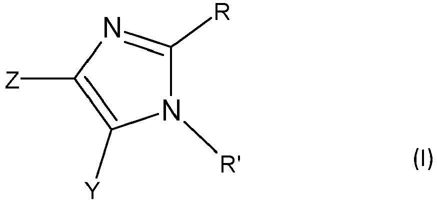 dans laquelle :
trois des quatre symboles Z, Y, R et R', identiques ou différents, représentent un atome d'hydrogène ou une chaîne carbonée pouvant contenir au moins un hétéroatome, Z et Y pouvant former ensemble avec les atomes de carbone auxquels ils se rattachent un cycle lorsque ni Z, ni Y ne désignent le 4^{ème} symbole,
et le seul quatrième symbole désigne un rattachement à l'élastomère diénique,
**caractérisé en ce que** les cycles imidazoles représentent entre 4 et 15 moles pour 100 moles d'unités monomères constituant l'élastomère diénique.

2. Elastomère diénique selon la revendication 1 dans lequel R' désigne le rattachement à l'élastomère.

3. Elastomère diénique selon la revendication 1 ou 2 dans lequel Z et Y sont chacun un atome d'hydrogène.

4. Elastomère diénique selon l'une quelconque des revendications 1 à 3 dans lequel R représente un atome d'hydrogène ou une chaîne carbonée pouvant contenir au moins un hétéroatome, de préférence un atome d'hydrogène ou un groupe alkyle.

5. Elastomère diénique selon l'une quelconque des revendications 1 à 4 dans lequel R contient 1 à 6 atomes de carbone, de préférence est un groupe méthyle.

6. Elastomère diénique selon l'une quelconque des revendications 1 à 5 dans lequel les cycles imidazoles représentent de 5 à 15 moles pour 100 moles d'unités monomères constituant l'élastomère diénique.

7. Elastomère diénique selon l'une quelconque des revendications 1 à 6, lequel élastomère diénique a pour unité diénique des unités monomères 1,3-butadiène ou 1,3-isoprène, de préférence 1,3-butadiène.

8. Elastomère diénique selon l'une quelconque des revendications 1 à 7 dans lequel les cycles imidazoles sont greffés sur les unités diéniques de l'élastomère diénique.

9. Elastomère diénique selon l'une quelconque des revendications 1 à 8 dans lequel les cycles imidazoles sont greffés sur l'élastomère diénique par réaction d'un composé 1,3-dipolaire et d'un élastomère diénique de départ, le composé 1,3-dipolaire de formule (II)
Q-A-B (II)
dans laquelle
Q comprend un dipôle contenant un atome d'azote,
A est un espaceur reliant Q et B,
B représente le cycle imidazole de formule (I) dans laquelle le quatrième symbole est rattaché directement à A.

10. Elastomère diénique selon la revendication 9 dans lequel le composé 1,3-dipolaire est choisi dans le groupe constitué par les oxydes de nitrile, les imines de nitrile et les nitrones.

11. Elastomère diénique selon l'une quelconque des revendications 9 à 10 dans lequel Q contient un motif -C≡N→O.

12. Elastomère diénique selon l'une quelconque des revendications 9 à 11 dans lequel le symbole Q comprend un noyau benzénique substitué par le dipôle oxyde de nitrile et également substitué par un ou plusieurs autres substituants dont un est en ortho du dipôle oxyde de nitrile.

13. Elastomère diénique selon l'une quelconque des revendications 9 à 12 dans lequel l'élastomère diénique de départ est choisi dans le groupe constitué par les copolymères de butadiène-styrène (SBR), les copolymères d'isoprène-butadiène (BIR), les copolymères d'isoprène-styrène (SIR), les copolymères d'isoprène-butadiène-styrène (SBIR) et les copolymères d'éthylène et de butadiène.

14. Composition de caoutchouc qui comprend une charge renforçante et l'élastomère diénique défini selon l'une quelconque des revendications 1 à 13.

15. Pneumatique qui comprend une bande de roulement, laquelle bande de roulement comprend une composition de caoutchouc définie selon la revendication 14.

## Patentansprüche

1. Dienelastomer, das seitenständige und entlang der Kette des Dienelastomers zufällig verteilte Imidazolringe trägt, 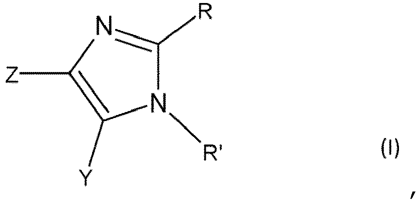 wobei:
drei der vier Symbole Z, Y, R und R' gleich oder verschieden sind und für ein Wasserstoffatom oder eine Kohlenstoffkette, die mindestens ein Heteroatom enthalten kann, stehen, wobei Z und Y dann, wenn weder Z noch Y das 4. Symbol bezeichnet, zusammen mit den Kohlenstoffatomen, an die sie gebunden sind, einen Ring bilden können,
und das einzige vierte Symbol eine Bindung an das Dienelastomer bezeichnet,
**dadurch gekennzeichnet, dass** die Imidazolringe zwischen 4 und 15 mol pro 100 mol Monomereinheiten, aus denen das Dienelastomer aufgebaut ist, ausmachen.

2. Dienelastomer nach Anspruch 1, wobei R' die Bindung an das Elastomer bezeichnet.

3. Dienelastomer nach Anspruch 1 oder 2, wobei Z und Y jeweils für ein Wasserstoffatom stehen.

4. Dienelastomer nach einem der Ansprüche 1 bis 3, wobei R für ein Wasserstoffatom oder eine Kohlenstoffkette, die mindestens ein Heteroatom enthalten kann, vorzugsweise ein Wasserstoffatom oder eine Alkylgruppe, steht.

5. Dienelastomer nach einem der Ansprüche 1 bis 4, wobei R 1 bis 6 Kohlenstoffatome enthält und vorzugsweise eine Methylgruppe ist.

6. Dienelastomer nach einem der Ansprüche 1 bis 5, wobei die Imidazolringe 5 bis 15 mol pro 100 mol Monomereinheiten, aus denen das Dienelastomer aufgebaut ist, ausmachen.

7. Dienelastomer nach einem der Ansprüche 1 bis 6, wobei das Dienelastomer als Dieneinheit 1,3-Butadien- oder 1,3-Isopren-Monomereinheiten, vorzugsweise 1,3-Butadien-Monomereinheiten, aufweist.

8. Dienelastomer nach einem der Ansprüche 1 bis 7, wobei die Imidazolringe auf die Dieneinheiten des Dienelastomers aufgepfropft sind.

9. Dienelastomer nach einem der Ansprüche 1 bis 8, wobei die Imidazolringe durch Reaktion einer 1,3-dipolaren Verbindung und eines Ausgangsdienelastomers auf das Dienelastomer aufgepfropft sind, wobei die 1,3-dipolare Verbindung der Formel (II) entspricht:
Q-A-B (II),
wobei
Q einen Dipol, der ein Stickstoffatom enthält, umfasst,
A ein Spacer, der Q mit B verbindet, ist,
B für den Imidazolring der Formel (I) steht, wobei das vierte Symbol direkt an A gebunden ist.

10. Dienelastomer nach Anspruch 9, wobei die 1,3-dipolare Verbindung aus der Gruppe bestehend aus Nitriloxiden, Nitriliminen und Nitronen ausgewählt ist.

11. Dienelastomer nach einem der Ansprüche 9 bis 10, wobei Q eine -C≡→O-Einheit enthält.

12. Dienelastomer nach einem der Ansprüche 9 bis 11, wobei das Symbol Q einen Benzolkern umfasst, der durch den Nitriloxid-Dipol substituiert ist und außerdem durch einen oder mehrere andere Substituenten substituiert ist, von denen einer in ortho-Position zum Nitriloxid-Dipol steht.

13. Dienelastomer nach einem der Ansprüche 9 bis 12, wobei das Ausgangsdienelastomer aus der Gruppe bestehend aus Butadien-Styrol-Copolymeren (SBR), Isopren-Butadien-Copolymeren (BIR), Isopren-Styrol-Copolymeren (SIR), Isopren-Butadien-Styrol-Copolymeren (SBIR) und Copolymeren von Ethylen und Butadien ausgewählt ist.

14. Kautschukzusammensetzung, die einen verstärkenden Füllstoff und das Dienelastomer gemäß einem der Ansprüche 1 bis 13 umfasst.

15. Reifen, der eine Lauffläche umfasst, wobei die Lauffläche eine Kautschukzusammensetzung gemäß Anspruch 14 umfasst.

## Claims

1. Diene elastomer which bears pendent imidazole rings of formula (1) distributed randomly along the chain of the diene elastomer, 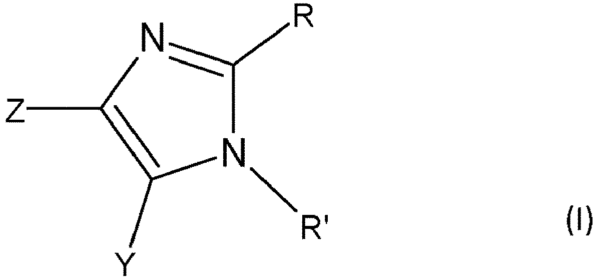 in which:
three of the four symbols Z, Y, R and R', which are identical or different, represent a hydrogen atom or a carbon chain that may contain at least one heteroatom, it being possible for Z and Y to form, together with the carbon atoms to which they are attached, a ring when neither Z nor Y denotes the 4^{th} symbol,
and solely the fourth symbol denotes an attachment to the diene elastomer,
**characterized in that** the imidazole rings represent between 4 and 15 mol per 100 mol of monomer units constituting the diene elastomer.

2. Diene elastomer according to Claim 1, in which R' denotes the attachment to the elastomer.

3. Diene elastomer according to Claim 1 or 2, in which Z and Y are each a hydrogen atom.

4. Diene elastomer according to any one of Claims 1 to 3, in which R represents a hydrogen atom or a carbon chain that may contain at least one heteroatom, preferably a hydrogen atom or an alkyl group.

5. Diene elastomer according to any one of Claims 1 to 4, in which R contains 1 to 6 carbon atoms, and preferably is a methyl group.

6. Diene elastomer according to any one of Claims 1 to 5, in which the imidazole rings represent from 5 to 15 mol per 100 mol of monomer units constituting the diene elastomer.

7. Diene elastomer according to any one of Claims 1 to 6, which diene elastomer has 1,3-butadiene or 1,3-isoprene monomer units, preferably 1,3-butadiene monomer units, as diene unit.

8. Diene elastomer according to any one of Claims 1 to 7, in which the imidazole rings are grafted onto the diene units of the diene elastomer.

9. Diene elastomer according to any one of Claims 1 to 8, in which the imidazole rings are grafted onto the diene elastomer by reacting a 1,3-dipolar compound and a starting diene elastomer, the 1,3-dipolar compound being of formula (II)
Q-A-B (II)
in which
Q comprises a dipole containing a nitrogen atom,
A is a spacer connecting Q and B,
B represents the imidazole ring of formula (I) in which the fourth symbol is attached directly to A.

10. Diene elastomer according to Claim 9, in which the 1,3-dipolar compound is selected from the group consisting of nitrile oxides, nitrile imines and nitrones.

11. Diene elastomer according to either one of Claims 9 and 10, in which Q contains a unit -C≡N→O.

12. Diene elastomer according to any one of Claims 9 to 11, in which the symbol Q comprises a benzene ring substituted with the nitrile oxide dipole and also substituted with one or more other substituents, one of which is in the position ortho to the nitrile oxide dipole.

13. Diene elastomer according to any one of Claims 9 to 12, in which the starting diene elastomer is selected from the group consisting of butadiene-styrene copolymers (SBRs), isoprene-butadiene copolymers (BIRs), isoprene-styrene copolymers (SIRs), isoprene-butadiene-styrene copolymers (SBIRs) and ethylene-butadiene copolymers.

14. Rubber composition which comprises a reinforcing filler and the diene elastomer defined according to any one of Claims 1 to 13.

15. Tyre which comprises a tread, which tread comprises a rubber composition defined according to Claim 14.
